(19) Europäisches Patentamt
European Patent Office
Office européen des brevets

(11) **EP 2 678 047 B1**

(12) **EUROPÄISCHE PATENTSCHRIFT**

(45) Veröffentlichungstag und Bekanntmachung des Hinweises auf die Patenterteilung:
**26.07.2017 Patentblatt 2017/30**

(21) Anmeldenummer: **12703744.8**

(22) Anmeldetag: **01.02.2012**

(51) Int Cl.:
*A61L 27/04* (2006.01)       *A61L 27/30* (2006.01)
*A61L 27/58* (2006.01)       *A61L 31/02* (2006.01)
*A61L 31/08* (2006.01)       *A61L 31/14* (2006.01)

(86) Internationale Anmeldenummer:
**PCT/EP2012/051669**

(87) Internationale Veröffentlichungsnummer:
**WO 2012/113624 (30.08.2012 Gazette 2012/35)**

(54) **IMPLANTAT AUS EINER BIOKORRODIERBAREN MAGNESIUMLEGIERUNG**

BIOCORRODIBLE MAGNESIUM ALLOY IMPLANT

IMPLANT EN ALLIAGE DE MAGNÉSIUM BIOCORRODABLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.02.2011 US 201161446051 P**

(43) Veröffentlichungstag der Anmeldung:
**01.01.2014 Patentblatt 2014/01**

(73) Patentinhaber: **Biotronik AG**
**8180 Bülach (CH)**

(72) Erfinder:
• **KALB, Hermann**
**91220 Schnaittach (DE)**
• **RZANY, Alexander**
**90449 Nürnberg (DE)**
• **GEROLD, Bodo**
**97753 Karlstadt (DE)**

(74) Vertreter: **Randoll, Sören et al**
**Biotronik SE & Co. KG**
**Corporate Intellectual Property**
**Woermannkehre 1**
**12359 Berlin (DE)**

(56) Entgegenhaltungen:
**EP-A2- 2 236 163       DE-A1-102006 038 238**
**US-A1- 2009 088 834**

**Beschreibung**

Die Erfindung betrifft ein Implantat mit einem Grundkörper aus einer biokorrodierbaren Magnesiumlegierung.

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate, z. B. Stents), zur Befestigung und temporären Fixierung von Gewebeimplantaten und Gewebstransplantationen, aber auch zu orthopädischen Zwecken, zum Beispiel als Nagel, Platte oder Schraube. Eine besonders häufig verwendete Form eines Implantats ist der Stent.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch A-neurysmenstents bekannt, die vorrangig zur Abdichtung des Aneuryrismas dienen. Die Stützfunktion ist zusätzlich gegeben.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Alternativ besitzen Form-Gedächtnis-Materialien wie Nitinol die Fähigkeit zur Selbstexpansion, wenn eine Rückstellkraft wegfällt, die das Implantat auf einem kleinen Durchmesser hält. Die Rückstellkraft wird in der Regel durch einen Schutzschlauch auf das Material ausgeübt.

Das Implantat, insbesondere der Stent, besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßem Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatwerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatwerkstoffs in verschiedener Weise. Entsprechend der Reaktion des Biosystems können die Implantatwerkstoffe in bioaktive, bioinerte und degradierbare bzw. resorbierbare Werkstoffe unterteilt werden.

Implantatwerkstoffe umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiAl6V4 oder Ti-A16Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen. Die vorliegende Erfindung befasst sich mit biokorrodierbaren Basislegierungen des Magnesiums.

Der Einsatz biokorrodierbarer Magnesiumlegierungen für temporäre Implantate mit filigranen Strukturen ist insbesondere dadurch erschwert, als dass die Degradation des Implantats in vivo sehr rasch verläuft. Um die Korrosionsrate, d. h. die Abbaugeschwindigkeit, zu mindern, sind verschiedene Ansätze in der Diskussion. Zum einen wird versucht, auf Seiten des Implantatwerkstoffs durch entsprechende Legierungsentwicklung die Degradation zu verlangsamen. Zum anderen sollen Beschichtungen eine temporäre Hemmung des Abbaus bewirken. Die bisherigen Ansätze sind zwar vielversprechend, sind jedoch allesamt bisher noch nicht in einem kommerziell erhältlichen Produkt verwirklicht. Vielmehr besteht ungeachtet der bisherigen Bemühungen nach wie vor ein anhaltender Bedarf nach Lösungsansätzen, die eine zumindest temporäre Herabsetzung der in vivo Korrosion von Magnesiumlegierungen ermöglichen.

US 2009/088834 beschreibt ein Stent mit einem biokorrodierbaren Grundkörper aus Magnesium, wobei das Magnesium eine Vielzahl von Hydroxylapatit-Partikeln enthält. Die Partikel verringern die Korrosionsgeschwindigkeits des Stentkörpers. Ein oder mehrere der zuvor angesprochenen Nachteile des Standes der Technik werden mit Hilfe des erfindungsgemäßen Implantats gelöst oder zumindest gemindert. Das erfindungsgemäße Implantat weist einen Grundkörper aus einer biokorrodierbaren Magnesiumlegierung auf. Die Magnesiumlegierung enthält eine Vielzahl von statistisch verteilten Partikeln, die aus einem oder mehreren der Elemente Y, Zr, Mn, Sc, Fe, Ni, Co, W, Pt und Seltene Erden der Ordnungszahlen 57 bis 71 bestehen oder aus Legierungen oder Verbindungen bestehen, die ein oder mehrere der genannten Elemente enthalten. Der mittlere Abstand der Partikel voneinander ist dabei kleiner als der hundertfache mittlere Partikeldurchmesser.

In der bisherigen Entwicklung von Magnesiumwerkstoffen wurde die Korrosionsfestigkeit immer durch eine Erhöhung der Reinheit des Magnesiumwerkstoffes verbessert. Als kritische Elemente gelten in diesem Zusammenhang Eisen, Nickel, Chrom und Kobalt. Partikel aus intermetallischen Verbindungen, Teilchen anderer chemischer Natur (Oxide, Hydride) bzw. Ausscheidungen (Al12Mg17) in Magnesiumwerkstoffen führen aufgrund des unterschiedlichen elektrochemischen Potentials zu mikrogalvanischer Korrosion. In deren Folge kommt es zu lokalen korrosiven Prozessen, welche die Korrosionsgeschwindigkeit des Werkstoffs massiv beschleunigen. Aus diesem Grund wurde bisher versucht, die Konzentration der Partikel möglichst gering halten.

Die erfinderische Lösung beschreitet hier jedoch genau den gegensätzlichen Weg. Beobachtet wird in Magnesiumwerkstoffen grundsätzlich eine lokal sehr inhomogen am Material angreifende Korrosion. Dabei treten kathodische Prozesse auf, welche mit der Freisetzung von Hydroxidionen und der Entwicklung von Wasserstoff einhergehen, und zwar an definierten Zentren, den oben genannten Partikeln. Der anodische Auflösungsprozess des Magnesiumwerkstoffs findet in der Umgebung des kathodischen Zentrums statt. Der Prozess lässt sich in folgende Teilreaktionen aufspalten:

$$\text{Anodisch:} \qquad Mg \rightarrow Mg^{2+} + 2\,e^-$$
$$\text{Kathodisch:} \qquad 2\,H_2O + 2\,e^- \rightarrow 2\,OH^- + H_2$$

Der anodische Prozess ist dabei stark pH-abhängig. So ist die Mg-Korrosion bei pH < 5 massiv beschleunigt und bei pH > 10 massiv verlangsamt und kommt praktisch vollständig zum Erliegen. Aufgrund dieses Verhaltens führt die Freisetzung von Hydroxidionen am kathodischen Zentrum zum Schutz der unmittelbaren Umgebung.

Der Erfindung liegt die Erkenntnis zugrunde, dass sich die Korrosion von Implantaten aus biokorrodierbaren Magnesiumlegierungen dadurch zeitlich hinauszögern lässt, dass eine Vielzahl homogen verteilter Partikel in das Werkstoffvolumen, einen oberflächennahen Bereich oder die Oberfläche eingetragen werden. Die Partikel wirken als kathodische Zentren im zuvor genannten Sinne, d.h. die Wasserstoffüberspannung ist hinreichend gering und die Reaktion kann mit hoher Rate ablaufen. Die Partikel bestehen aus einem oder mehreren der Elemente Y, Zr, Mn, Sc, Fe, Ni, Co, W, Pt und Seltene Erden der Ordnungszahlen 57 bis 71 oder aus Legierungen oder Verbindungen, die ein oder mehrere der genannten Elemente enthalten. Unter dem Begriff Legierung fallen vorliegend metallische Zusammensetzungen der Elemente, aber auch Zusammensetzungen, bei denen kovalente Bindungen zwischen den Elementen bestehen. Die Legierungen enthalten vorzugsweise Magnesium. Verbindungen umfassen insbesondere Hydride und Carbide der genannten Elemente.

Als biokorrodierbar im Sinne der Erfindung werden Legierungen bezeichnet, bei denen in physiologischer Umgebung ein Abbau bzw. Umbau stattfindet, so dass der aus dem Werkstoff bestehende Teil des Implantates ganz oder zumindest überwiegend nicht mehr vorhanden ist.

Unter Magnesiumlegierung wird vorliegend ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Die Legierung ist so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6.8 g/l, CaCl$_2$ 0.2 g/l, KCl 0.4 g/l, MgSO$_4$ 0.1 g/l, NaHCO$_3$ 2.2 g/l, Na$_2$HPO$_4$ 0.126 g/l, NaH$_2$PO$_4$ 0.026 g/l). Eine Probe der zu untersuchenden Legierung wird dazu in einem verschlossenen Probenbehälter mit einer definierten Menge des Prüfmediums bei 37°C und pH 7.38 gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Der Begriff Korrosion bezieht sich vorliegend auf die Reaktion eines metallischen Werkstoffes mit seiner Umgebung, wobei eine messbare Veränderung des Werkstoffs bewirkt wird, die - bei Einsatz des Werkstoffs in einem Bauteil - zu einer Beeinträchtigung der Funktion des Bauteils führt. Der Vorgang der Korrosion lässt sich durch Angabe einer Korrosionsrate quantifizieren. Ein zügiger Abbau ist mit einer hohen Korrosionsrate verbunden, und umgekehrt. Bezogen auf den Abbau des gesamten Grundkörpers wird ein im Sinne der Erfindung modifiziertes Implantat zur Herabsetzung der Korrosionsrate führen. Die Partikel haben vorzugsweise einen mittleren Durchmesser von 1 Nanometer bis 10 Mikrometer, besonders bevorzugt von 500 Nanometer bis 3 Mikrometer, insbesondere von 1 bis 2 Mikrometer.

In der Umgebung des kathodischen Zentrums bilden sich infolge der Freisetzung von Hydroxidionen geschützte Bereiche aus. Die Größe des geschützten Bereichs um ein einzelnes kathodisches Zentrum hängt von Größe und Zusammensetzung der Partikel sowie der umgebenden Matrix des Magnesiumwerkstoffes ab. Die Größe der geschützten Fläche pro Partikel sollte mindestens 1 Quadratmikrometer, bevorzugt bis zu 100 Quadratmikrometer, insbesondere bevorzugt bis zu 10000 Quadratmikrometer betragen.

Innerhalb des Werkstoffs weist die Fläche der geschützten Bereiche eine Größenverteilung auf, welche von der Verteilung der Partikel bestimmt wird. Die Schutzwirkung auf die Gesamtoberfläche des Magnesiumwerkstoffs ist abhängig von Anzahl und Größenverteilung der geschützten Bereiche. Vorzugsweise beträgt die Anzahl der Partikel auf der Oberfläche des Grundkörpers 1*10^2 bis 1*10^6 Partikel pro mm$^2$ bzw. beträgt die Anzahl der Partikel im Volumen des Grundkörpers 1*10^3 bis 1*10^9 Partikel pro mm$^3$. Ein Verhältnis des mittleren Partikeldurchmesser zum mittleren Abstand der Partikel voneinander liegt vorzugsweise im Bereich von 1:2 bis 1:100, insbesondere 1:2 bis 1:10.

Die Korrosionsrate wird wie folgt durch die kathodischen Zentren quantitativ beeinflusst:

a) Die geschützte Gesamtfläche $A\_protect$ ergibt sich unter Annahme nicht überlappender Schutzbereiche aus der Summe über die Verteilung der von den einzelnen kathodischen Zentren geschützten Flächen $A\_cathodic\_center$:

$$A_{protect} = \sum_{i=1\cdots N} A_i^{cathodic\ center}$$

b) Die Korrosionsrate $R\_corr$ ist direkt proportional zu der Korrosion zugänglichen Probenfläche $A\_corr$, wobei $A\_total$ die Gesamtfläche des Materials bezeichnet:

$$R_{corr} \propto A_{corr} \propto A_{total} - A_{protect} \propto A_{total}\left(1 - \frac{A_{protect}}{A_{total}}\right)$$

Die Korrosionsrate nimmt bei Annahme gleicher Abtragtiefe damit bei höherem Flächenanteil der geschützten Bereiche ab. Die genannten Flächenanteile lassen sich experimentell bestimmen.

Eine besonders große Schutzwirkung wird genau dann erreicht, wenn hinreichend viele kathodische Zentren gleichmäßig im Werkstoff verteilt sind und der Überlapp zwischen den geschützten Bereichen möglichst gering ist. Der optimale mittlere Abstand $d\_mean$ zwischen kathodischen Zentren ohne Überlapp lässt sich aus einer statistischen Betrachtung der Verteilung abschätzen:

$$d_{mean} = 2 \cdot \sqrt{\frac{A_{protect}}{N \cdot \pi}}$$

Die Schutzwirkung lässt sich also sowohl durch viele kleine geschützte Bereiche als auch wenig große geschützte Bereiche erhöhen. Vorzugsweise liegt der mittlere Abstand der Partikel im Bereich von 200 nm bis 100 μm. Insbesondere ist der mittlere Abstand kleiner als 20 μm.

Die geschützte Fläche pro kathodisches Zentrum ist abhängig von der chemischen Natur des kathodischen Zentrums und der Werkstoffmatrix.

Die erfinderische Modifikation des Werkstoffs lässt sich nicht nur auf das gesamte Werkstoffvolumen anwenden, sondern ist optional auf die Oberfläche oder den oberflächennahen Bereich eines Implantates beschränkbar. So ist es möglich kathodische Zentren gezielt durch Walzen in die Oberfläche eines Werkstücks einzubringen. Dadurch wird eine initiale Korrosionsbarriere geschaffen und die Abbaurate nimmt mit der Zeit zu. Vorzugsweise sind die Partikel in die Oberfläche oder den oberflächennahen Bereich des Grundkörpers eingearbeitet. Es ergibt sich dann zu Beginn der einsetzenden korrosiven Prozesse eine relativ niedrige Korrosionsrate, die im Laufe der Zeit ansteigt. Dieses Verhalten wird als temporäre Herabsetzung der Korrosionsrate bezeichnet. Im Falle von Koronarstents soll die mechanische Integrität der Struktur über einen Zeitraum von drei bis sechs Monaten nach Implantation aufrechterhalten werden.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren. Das Implantat besteht ganz oder in Teilen aus dem biokorrodierbaren Werkstoff. Wenn das Implantat nur in Teilen aus dem biokorrodierbaren Werkstoff besteht, so ist dieser Teil entsprechend zu modifizieren. Vorzugsweise ist das Implantat ein Stent.

Ein weiterer Aspekt der Erfindung liegt in der Bereitstellung zweier Verfahren zur Herstellung eines Implantats mit einem Grundkörper aus einer biokorrodierbaren Magnesiumlegierung, wobei die Magnesiumlegierung eine Vielzahl von statistisch verteilten Partikeln oben genannter Zusammensetzung enthält und der mittlere Abstand der Partikel voneinander kleiner als der hundertfache mittlere Partikeldurchmesser ist und die Partikel in die Oberfläche oder in einem oberflächennahen Bereich des Grundkörpers eingearbeitet sind.

Nach einer ersten Variante umfasst das Verfahren die Schritte:

(i) Bereitstellen eines Rohlings aus der biokorrodierbaren Magnesiumlegierung;
(ii) Auftragen einer nicht-wässrigen Suspension von Partikeln der genannten Zusammensetzung auf dem Rohling; und
(iii) Einwalzen der Partikel in die Oberfläche oder in den oberflächennahen Bereich des Rohlings.

Demnach wird eine ölige Suspension, die die einzuarbeitenden Partikel enthält, auf den Rohling, aus dem der Grundkörper geformt werden soll, aufgebracht und durch Walzen eingearbeitet. Sowohl beim Kaltwalzen als auch beim Warmwalzen lässt sich diese Suspension als Schmiermittel einsetzen. Durch Optimierung der Parameter Volumenstrom der Suspension, Temperatur, Anpressdruck, Geschwindigkeit lässt sich die Einarbeitung der Partikel in die Oberfläche des gewalzten Magnesiumwerkstoffs optimieren. Die Variante eignet sich insbesondere für Magnesiumlegierungen auf Basis von WE43.

Nach einer zweiten Variante umfasst das Verfahren die Schritte:

(i) Bereitstellen eines Rohlings aus der biokorrodierbaren Magnesiumlegierung;
(ii) Auftragen von Partikeln der genannten Zusammensetzung auf den Rohling; und
(iii) Aufschmelzen der Magnesiumlegierung im oberflächennahen Bereich des Rohlings.

Gemäß dieser Variante werden die einzuarbeitenden Partikel direkt auf den Rohling aufgebracht, der später den Grundkörper bilden soll. Anschließend wird die Magnesiumlegierung an der Oberfläche zum Beispiel durch Laserbehandlung lokal aufgeschmolzen. Nach dem Abkühlen sind die Partikel dann in dem oberflächennahen Bereich des Rohlings eingebettet.

Die Erfindung wird nachfolgend anhand von Ausführungsbeispielen näher erläutert.

Ausführungsbeispiel 1

Auf einem plattenförmigen Rohling aus der Magnesiumlegierung AZ31 wird eine EisenPartikel (Chemikalien zur Herstellung erhältlich bei Sigma-Aldrich, Partikeldurchmesser kleiner 100 nm) enthaltende Suspension z. B. durch Sprühen oder Tauchen aufgetragen, um einen Film mit einer statistisch homogenen Verteilung an Eisen-Partikeln zu erzeugen.

Sowohl beim Kaltwalzen als auch beim Warmwalzen lässt sich diese Suspension als Schmiermittel einsetzen. Durch den Walzprozess werden die Partikel in die Oberfläche des Rohlings eingearbeitet. Die Partikel erhöhen dabei nicht nur den Korrosionsschutz, sondern auch die Verschleißbeständigkeit durch Erhöhung der Härte. Der Rohling wird nachfolgend zum Grundkörper des Implantats weiterverarbeitet.

Ausführungsbeispiel 2

Auf einem plattenförmigen Rohling aus der Magnesiumlegierung AZ31 werden WolframPartikel (erhältlich bei Sigma-Aldrich; Partikeldurchmesser ca. 150 nm) als Pulver aufgetragen und durch Rütteln homogen verteilt. Bei komplizierten dreidimensionalen Strukturen bietet sich auch die Verwendung eines haftvermittelnden Polymers zu Beschichtung der Oberfläche vor dem Laser-Legieren an. Durch Variation des Verhältnisses Polymer/Wolframpartikel lässt sich unmittelbar der mittlere Abstand zwischen Wolframpartikeln einstellen.

Die Einarbeitung der Wolframpartikel in die Magnesiumlegierung erfolgt durch Laser-Legieren. Dazu wird das Werkstück mit einer Hochleistungslaserdiode unter Argon-Schutzgas lokal aufgeschmolzen. Die Laserleistung beträgt dabei zwischen 1.2 und 1.6 kW, die Vorschubgeschwindigkeit des Lasers 0.5 bis 1.0 m/min. Durch das Argon wird eine Oxidation des Magnesiumwerkstoffs und des Wolfram während der Bearbeitung verhindert.

Die Verwendung der Technologie des Laser-Legierens erlaubt insbesondere den lokalen Schutz eines Werkstücks aus einer Magnesiumlegierung. Im Zusammenhang mit Stents kann zum Beispiel durch die lokale Beeinflussung der Degradationsrate eine sequentielle Fragmentierung des Implantats erreicht werden, etwa indem die Oberflächen der Segmentringe einer Stentstruktur mit erfindungsgemäßen kathodischen Zentren versehen werden, die longitudinalen Verbindungsstege der Segmentringe jedoch nicht, wodurch diese schneller degradieren, als die Segmentringe. Durch das schnellere Auflösen der Verbindungsstege wird rasch eine hohe longitudinale Flexibilität erreicht, wobei die Tragkraft der Segmentringe noch erhalten bleibt.

Die Partikel dienen dabei nicht nur dem Korrosionsschutz sondern auch zur Erhöhung der Verschleißbeständigkeit gegen Abrasion durch Erhöhung der Härte. Zudem kann durch geeignete Wahl der Partikel und ihrer Zusammensetzung eine wirkungsvolle Anbindung von polymeren Substanzen an die Oberfläche erfolgen. Diese polymeren Substanzen können einerseits eine korrosionshemmende Wirkung haben, andererseits können sie eine oder mehrere pharmakologische Wirkstoffe enthalten oder selbst eine pharmakologische Wirkung besitzen.

Die zusätzliche Beschichtung mit einem Polymer lässt sich beispielsweise technisch wie folgt realisieren. PLLA L214S (Boehringer Ingelheim) wird in einer Konzentration von 1.6% (w/v) in Chloroform gelöst und Rapamycin als Wirksubstanz zugeben. Der Wirkstoffanteil liegt dabei vorzugsweise zwischen 15% und 20% bezogen auf den Feststoffanteil. Das Implantat aus der modifizierten Magnesiumlegierung wird mit Hilfe eines Tauchroboters für 1 Sekunde in die Lösung getaucht, herausgezogen und an der Luft mit Stickstoff angeblasen um das Lösungsmittel zu verdampfen. Dieser Vorgang wird so oft wiederholt, bis eine ausreichende Schichtdicke von etwa 5 µm erreicht wird.

Die Ausführungsbeispiele gelten analog auch für andere biokorrodierbare Magnesiumlegierungen und Partikelzusammensetzungen.

## Patentansprüche

1. Implantat mit einem Grundkörper aus einer biokorrodierbaren Magnesiumlegierung, wobei die Magnesiumlegierung eine Vielzahl von statistisch verteilten Partikeln enthält, wobei der mittlere Abstand der Partikel voneinander kleiner als der hundertfache mittlere Partikeldurchmesser ist und die Partikel aus einem oder mehreren der Elemente Y, Zr, Mn, Sc, Fe, Ni, Co, W, Pt und Seltene Erden der Ordnungszahlen 57 bis 71 bestehen oder aus Legierungen oder Verbindungen bestehen, die ein oder mehrere der genannten Elemente enthalten.

2. Implantat nach Anspruch 1, bei dem die Partikel einen mittleren Durchmesser von 1 nm bis 10 µm aufweisen.

3. Implantat nach Anspruch 1 oder 2, bei dem die Partikel in eine Oberfläche oder in einem oberflächennahen Bereich des Grundkörpers eingearbeitet sind.

4. Implantat nach einem der vorhergehenden Ansprüche, bei dem eine Anzahl der Partikel auf der Oberfläche des Grundkörpers im Bereich von $1*10^2$ bis $1*10^6$ pro mm$^2$ liegt.

5. Implantat nach einem der vorhergehenden Ansprüche, bei dem eine Anzahl der Partikel im Volumen des Grundkörpers im Bereich von $1*10^3$ bis $1*10^9$ pro mm$^3$ liegt.

6. Implantat nach Anspruch 4 oder 5, bei dem ein Verhältnis des mittleren Partikeldurchmesser zum mittleren Abstand der Partikel voneinander im Bereich von 1:2 bis 1:10 liegt.

7. Implantat nach einem der vorhergehenden Ansprüche, bei dem ein mittlerer Abstand der Partikel im Bereich von 200 nm bis 100 µm liegt.

8. Implantat nach einem der vorhergehenden Ansprüche, bei dem das Implantat ein Stent ist.

9. Verfahren zur Herstellung eines Implantats mit einem Grundkörper aus einer biokorrodierbaren Magnesiumlegierung, wobei die Magnesiumlegierung eine Vielzahl von statistisch verteilten Partikeln enthält, wobei der mittlere Abstand der Partikel voneinander kleiner als der hundertfache mittlere Partikeldurchmesser ist und die Partikel aus einem oder mehreren der Elemente Y, Zr, Mn, Sc, Fe, Ni, Co, W, Pt und Seltene Erden der Ordnungszahlen 57 bis 71 bestehen oder aus Legierungen oder Verbindungen bestehen, die ein oder mehrere der genannten Elemente enthalten, und die Partikel in eine Oberfläche oder in einem oberflächennahen Bereich des Grundkörpers eingearbeitet sind, wobei das Verfahren die Schritte umfasst:

   (i) Bereitstellen eines Rohlings aus der biokorrodierbaren Magnesiumlegierung;
   (ii) Auftragen einer nicht-wässrigen Suspension von Partikeln der genannten Zusammensetzung auf den Rohling; und
   (iii) Einwalzen der Partikel in die Oberfläche oder in den oberflächennahen Bereich des Rohlings.

10. Verfahren zur Herstellung eines Implantats mit einem Grundkörper aus einer biokorrodierbaren Magnesiumlegierung, wobei die Magnesiumlegierung eine Vielzahl von statistisch verteilten Partikeln enthält, wobei der mittlere Abstand der Partikel voneinander kleiner als der hundertfache mittlere Partikeldurchmesser ist und die Partikel aus einem oder mehreren der Elemente Y, Zr, Mn, Sc, Fe, Ni, Co, W, Pt und Seltene Erden der Ordnungszahlen 57 bis 71 bestehen oder aus Legierungen oder Verbindungen bestehen, die ein oder mehrere der genannten Elemente enthalten, und die Partikel in eine Oberfläche oder in einem oberflächennahen Bereich des Grundkörpers eingearbeitet sind, wobei das Verfahren die Schritte umfasst:

(i) Bereitstellen eines Rohlings aus der biokorrodierbaren Magnesiumlegierung;
(ii) Auftragen von Partikeln der genannten Zusammensetzung auf den Rohling; und
(iii) Aufschmelzen der Magnesiumlegierung im oberflächennahen Bereich des Rohlings.

## Claims

1. An implant with a base body made of a biocorrodible magnesium alloy, the magnesium alloy containing many statistically distributed particles that have a mean distance from one another that is less than a hundred times the mean particle diameter and that consist of one or more of the elements Y, Zr, Mn, Sc, Fe, Ni, Co, W, Pt, and the rare earths having atomic numbers 57 through 71, or of alloys or compounds containing one or more of the mentioned elements.

2. An implant according to claim 1, wherein the particles have a mean diameter of 1 nm to 10 $\mu$m.

3. An implant according to claim 1 or 2, wherein the particles are incorporated into the surface of the base body, or into an area that is near its surface.

4. An implant according to any one of the preceding claims, wherein the number of particles on the surface of the base body lies in the range from $1*10^2$ to $1*10^6$ particles per mm$^2$.

5. An implant according to any one of the preceding claims, wherein the number of particles in the volume of the base body is in the range from $1*10^3$ to $1*10^9$ particles per mm$^3$.

6. An implant according to claim 4 or 5, wherein the ratio of the mean particle diameter to the mean distance of the particles from one another lies in the range from 1:2 to 1:10.

7. An implant according to any one of the preceding claims, wherein the mean distance of particles lies in the range from 200 nm to 100 $\mu$m.

8. An implant according to any one of the preceding claims, wherein the implant is a stent.

9. A process for producing an implant with a base body made of a biocorrodible magnesium alloy, the magnesium alloy containing many statistically distributed particles that have a mean distance from one another that is less than a hundred times the mean particle diameter and that consist of one or more of the elements Y, Zr, Mn, Sc, Fe, Ni, Co, W, Pt, and the rare earths having atomic numbers 57 through 71, or of alloys or compounds containing one or more of the mentioned elements, and that are incorporated into the surface of the base body, or into an area that is near its surface, this process comprising the steps:

(i) Preparing of a blank of the biocorrodible magnesium alloy;
(ii) Applying a non-aqueous suspension of particles of the mentioned composition onto the blank; and
(iii) Rolling the particles into the surface of the blank, or into the area near its surface.

10. A process for producing an implant with a base body made of a biocorrodible magnesium alloy, the magnesium alloy containing many statistically distributed particles that have a mean distance from one another that is less than a hundred times the mean particle diameter and that consist of one or more of the elements Y, Zr, Mn, Sc, Fe, Ni, Co, W, Pt, and the rare earths having atomic numbers 57 through 71, or of alloys or compounds containing one or more of the mentioned elements, and that are incorporated into the surface of the base body, or into an area that is near its surface, this process comprising the steps:

(i) Preparing of a blank of the biocorrodible magnesium alloy;
(ii) Applying particles of the mentioned composition onto the blank; and
(iii) Fusing the magnesium alloy in the area of the blank that is near the surface.

**Revendications**

1. Implant avec un corps de base constitué d'un alliage de magnésium biocorrodable, où l'alliage de magnésium contient un grand nombre de particules distribuées de manière statistique, où la distance moyenne des particules entre elles est inférieure à un centième du diamètre particulaire moyen et les particules sont constituées d'un ou de plusieurs éléments parmi l'Y, le Zr, le Mn, le Sc, le Fe, le Ni, le Co, le W, le Pt et les terres rares de numéros 57 à 71 ou sont constituées d'alliages ou de composés qui contiennent un ou plusieurs parmi les éléments cités.

2. Implant selon la revendication 1, chez lequel les particules présentent un diamètre moyen de 1 nm à 10 $\mu$m.

3. Implant selon la revendication 1 ou 2, chez lequel les particules sont incorporées dans une surface ou dans une région du corps de base proche de la surface.

4. Implant selon l'une des revendications précédentes, chez lequel un nombre de particules se situe sur la surface du cops de base dans la région de 1*10^2 à 1*10^6 par mm$^2$.

5. Implant selon l'une des revendications précédentes, chez lequel un nombre de particules se situe dans le volume du cops de base dans la région de 1*10^3 à 1*10^9 par mm$^3$.

6. Implant selon la revendication 4 ou 5, chez lequel un rapport du diamètre particulaire moyen sur une distance moyenne des particules entre elles se situe dans la plage de 1:2 à 1:10.

7. Implant selon l'une des revendications précédentes, chez lequel une distance moyenne des particules se situe dans la plage de 200 nm à 100 $\mu$m.

8. Implant selon l'une des revendications précédentes, chez lequel l'implant est un stent.

9. Procédé de fabrication d'un implant avec un corps de base constitué d'un alliage de magnésium biocorrodable, où l'alliage de magnésium contient un grand nombre de particules distribuées de manière statistique, où la distance moyenne des particules entre elles est inférieure à un centième du diamètre particulaire moyen et les particules sont constituées d'un ou de plusieurs éléments parmi l'Y, le Zr, le Mn, le SC, le Fe, le Ni, le Co, le W, le Pt et les terres rares de numéros 57 à 71 ou sont constituées d'alliages ou de composés qui contiennent un ou plusieurs parmi les éléments cités, et les particules sont incorporées dans une surface ou dans une région du corps de base proche de la surface, où le procédé comprend les étapes :

   (i) de mise à disposition d'une pièce brute constituée d'un alliage de magnésium biocorrodable ;
   (ii) de dépôt d'une suspension non aqueuse de particules de ladite composition sur la pièce brute, et
   (iii) de pressage par rouleaux des particules dans la surface ou dans la région proche de la surface de la pièce brute.

10. Procédé de fabrication d'un implant avec un corps de base constitué d'un alliage de magnésium biocorrodable, où l'alliage de magnésium contient un grand nombre de particules distribuées de manière statistique, où la distance moyenne des particules entre elles est inférieure à un centième du diamètre particulaire moyen et les particules sont constituées d'un ou de plusieurs éléments parmi l'Y, le Zr, le Mn, le SC, le Fe, le Ni, le Co, le W, le Pt et les terres rares de numéros 57 à 71 ou sont constituées d'alliages ou de composés qui contiennent un ou plusieurs parmi les éléments cités, et les particules sont incorporées dans une surface ou dans une région du corps de base proche de la surface, où le procédé comprend les étapes :

   (i) de mise à disposition d'une pièce brute constituée d'un alliage de magnésium biocorrodable ;
   (ii) de dépôt de particules de ladite composition sur la pièce brute, et
   (iii) de fusion de l'alliage de magnésium dans la région proche de la surface de la pièce brute.

**EP 2 678 047 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2009088834 A